Europäisches Patentamt

European Patent Office

Office européen des brevets

(19)

(11) **EP 0 731 913 B1**

(12) **EUROPÄISCHE PATENTSCHRIFT**

(45) Veröffentlichungstag und Bekanntmachung des
Hinweises auf die Patenterteilung:
**10.03.1999 Patentblatt 1999/10**

(21) Anmeldenummer: **95903264.0**

(22) Anmeldetag: **21.11.1994**

(51) Int Cl.⁶: **G01N 33/34**, D21G 9/00

(86) Internationale Anmeldenummer:
**PCT/EP94/03843**

(87) Internationale Veröffentlichungsnummer:
**WO 95/15492 (08.06.1995 Gazette 1995/24)**

(54) **VORRICHTUNG ZUR UNTERSUCHUNG DES QUERPROFILS EINER KONTINUIERLICH ERZEUGTEN MATERIALBAHN**

DEVICE FOR MONITORING THE CROSS-SECTIONAL SHAPE OF A CONTINUOUSLY PRODUCED WEB OF MATERIAL

DISPOSITIF PERMETTANT D'EXAMINER LE PROFIL TRANSVERSAL D'UNE BANDE DE MATERIAU PRODUITE EN CONTINU

(84) Benannte Vertragsstaaten:
**DE SE**

(30) Priorität: **02.12.1993 EP 93119465**

(43) Veröffentlichungstag der Anmeldung:
**18.09.1996 Patentblatt 1996/38**

(73) Patentinhaber: **SIEMENS AKTIENGESELLSCHAFT**
**80333 München (DE)**

(72) Erfinder: **ADAMY, Jürgen**
**D-91338 Igensdorf (DE)**

(56) Entgegenhaltungen:
**EP-A- 0 351 260          US-A- 4 874 467**
**US-A- 5 170 357**

**Beschreibung**

Die Erfindung betrifft ein Verfahren zur Untersuchung des Querprofils einer kontinuierlich erzeugten Materialbahn, wobei für eine Querprofilregelung mit einer Regeleinrichtung, der eingangsseitig die Regelabweichung zwischen einem vorgegebenen Sollprofil und dem gemessenen Profil der Materialbahn zuführbar ist und die ausgangsseitig in Abhängigkeit von der Regelabweichung eine vorgegebene Anzahl von Stellgliedern zum Eintellen des Profils der Materialbahn betätigen kann, in einer Recheneinrichtung in Abhängigkeit von einem mathematischen Modell der Regelstrecke das Sollprofil berechenbar ist wird. Eine zugehörige Vorrichtung mit einer Regeleinrichtung ist im einzelnen aus der US-A-5 170 357 vorbekannt, bei der es um die Optimierung der eigentlichen Querprofilregelung geht.

Bei vielen Prozessen, mit denen kontinuierlich Materialbahnen erzeugt werden, ist es von Interesse, daß das Produkt, beispielsweise Papierbahnen oder Folien, möglichst überall gleiche Eigenschaften aufweist; es soll also homogen und von gleichbleibender Qualität sein. Um die jeweils interessierende Materialeigenschaft, wie z.B. Dicke, spezifisches Gewicht oder Feuchtegehalt, zahlenmäßig erfassen zu können, wird sie in Längs- und Querrichtung der Materialbahn gemessen und in diesen Richtungen als Profil dargestellt. Das Querprofil wird dabei im allgemeinen aufgelöst in einer Anzahl von Meßwerten ermittelt, wobei das ermittelte Profil mittelwertfrei ist oder mittelwertfrei gemacht wird. Wunsch ist es, daß das gemessene Profil einem vorgegebenen Sollprofil entspricht. Oftmals ist dieses Sollprofil einfach überall Null. Um dies zu erreichen, ist quer zur Materialbahn eine vorgegebene Anzahl von Stellgliedern angebracht, die von einer Regeleinrichtung in Abhängigkeit von der Regelabweichung zwischen dem vorgegebenen Sollprofil und dem gemessenen Profil betätigt werden.

Es ist plausibel, daß man mit einer endlichen Zahl von Stellgliedern einem Querprofil nicht jede beliebige Form geben kann. Insbesondere wird man keine völlige Glattheit erzielen können. Damit ergibt sich die Frage, wie weit durch die Regelung des Querprofils dieses überhaupt an das gewünschte Sollprofil angenähert werden kann.

Aus R. Münch: "Der JETCOmmander - ein fortschrittliches System zur Fernverstellung der Stoffauflaufblende und zur Regelung des Flächengewicht-Querprofils", Wochenblatt für Papierfabrikation 7, 1992, Seiten 259 - 265, ist ein Verfahren zur Abschätzung des Verbesserungspotentials bei einer Regelung des Flächengewicht-Querprofils an einer Papiermaschine bekannt, wobei die Varianz des gemessenen Profils in ihre Wellenlängenanteile zerlegt wird und daraus das Verbesserungspotential für das gemessene Profil abgeschätzt wird. Dabei ist die Genauigkeit der Abschätzung dadurch begrenzt, daß das optimal erreichbare Profil nicht bekannt ist.

Aufgabe der Erfindung ist es demgegenüber, Verfahren anzugeben, welche die Beurteilung von Regeleinrichtungen für Querprofile kontinuierlich erzeugter Materialbahnen verbessern.

Die Aufgabe ist erfindungsgemäß durch die Gesamtheit der im Patentanspruch 1 angegebenen Verfahrensmerkmale gelöst.

Weiterbildungen des Verfahrens sind in den abhängigen Ansprüchen angegeben.

Der wesentliche Vorteil des erfindungsgemäßen Verfahrens besteht darin, daß zunächst das eigentlich gesuchte, optimal erreichbare Profil bestimmt wird. Dieses Profil ist, wie später noch erläutert wird, keine Konstante, sondern abhängig vom jeweils gemessenen Profil. Es liefert, verglichen mit dem gemessenen Profil, ein sehr anschauliches Beurteilungsinstrument für die Abschätzung der möglichen Profilverbesserung.

Zur näheren Erläuterung der Erfindung wird im folgenden auf die Figuren der Zeichnung Bezug genommen; im einzelnen zeigen:

FIG 1        ein allgemeines Blockschaltbild einer zur Durchführung des erfindungsgemäßen Verfahrens geeigneten Vorrichtung,

FIG 2        die Grundstruktur eines Regelkreises zur Querprofilregelung,

FIG 3-6      Beispiele für gemessene und zugehörige, entsprechend dem erfindungsgemäßen Verfahren bestimmte, optimal erreichbare Profile,

FIG 7-10     die Fourierspektren der Profile in den FIG 3 bis 6 und

FIG 11       den Verlauf einer Grenzkurve, die angibt, wie gut das Spektrum des optimal erreichbaren Profils mindestens ist.

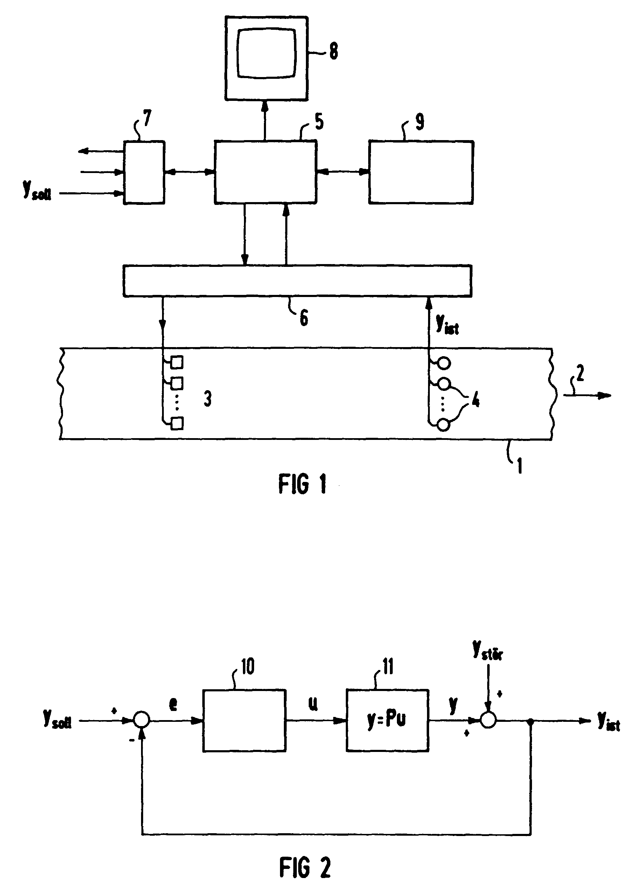

FIG 1 zeigt eine Vorrichtung zur Regelung des Querprofils einer kontinuierlich erzeugten Materialbahn 1, wobei der zu regelnde Prozeß, also die Erzeugung eines vorgegebenen Querprofils, in Richtung des Pfeiles 2 abläuft. Zur Einstellung des Querprofils der Materialbahn 1 ist eine vorgegebene Anzahl von n Stellgliedern 3 vorzugsweise äquidistant über die gesamte Breite der Materialbahn 1 angeordnet. In Richtung 2 des Prozeßablaufes gesehen ist hinter den Stellgliedern 3 eine weitere Anzahl von m Meßwertaufnehmern 4 angeordnet, die das Querprofil der Materialbahn 1, aufgelöst in m Meßwerte, erfassen. Zur Regelung des Querprofils der Materialbahn 1 dient eine Recheneinrichtung 5, die aus einem Digitalrechner mit Arbeits-, Programm- und Datenspeicher besteht. Zwischen der Recheneinrichtung 5 und dem Prozeß ist ein Schnittstellenbaustein 6 vorgesehen. Die Recheneinrichtung 5 erzeugt gemäß einem in ihr ablaufenden Regelungsprogramm Stellbefehle für die Stellglieder 3, die diesen über den Schnittstellenbaustein 6 zugeführt werden. Die Erzeugung der Stellbefehle für die Stellglieder 3 erfolgt dabei in Abhängigkeit von der Regelabweichung zwischen dem mit den Meßwertaufnehmern 4 erfaßten und der Recheneinrichtung 5 über den Schnittstellenbaustein 6 zugeführten Istprofil $\mathbf{y}_{ist}$ und einem vorgegebenen Sollprofil $\mathbf{y}_{soll}$. Dieses wird der Recheneinrichtung 5 über eine Ein-/Ausgabeeinheit 7 zugeführt, über die die Recheneinrichtung 5 mit der Außenwelt kommuniziert. An der Recheneinrichtung 5 ist ferner eine Anzeigevorrichtung 8 zur Visualisierung des Prozeßgeschehens angeschlossen. Ferner steht die Recheneinrichtung 5 mit einer separaten Einrichtung 9 in Verbindung, bei der es sich entweder um eine weitere Recheneinrichtung oder um ein zum Ablauf in der Recheneinrichtung 5 bestimmtes Programm zur Durchführung des im folgenden weiter erläuterten erfindungsgemäßen Verfahrens handelt.

FIG 2 zeigt die Grundstruktur des von der in FIG 1 gezeigten Vorrichtung gebildeten Querprofil-Regelkreises. Dieser besteht aus einem Regler 10 und einer Regelstrecke 11. Der Regler 10 ist in Form des in der Recheneinrichtung 5 ablaufenden Regelungsprogrammes realisiert und die Regelstrecke 11 wird von dem zu regelnden Prozeß, d. h. der Einstellung des Querprofils der Materialbahn 1 gebildet. Der Regler 10 erzeugt in Abhängigkeit von der Regelabweichung $\mathbf{e}$ zwischen dem Sollprofil $\mathbf{y}_{soll}$ und dem gemessenen Istprofil $\mathbf{y}_{ist}$ die auf den Prozeß über die Stellglieder 3 einwirkende Stellgröße bzw. das Stellprofil $\mathbf{u}$. Entsprechend der Charakteristik des zu regelnden Prozesses wird in der Regelstrecke 11 aus dem Stellprofil $\mathbf{u}$ das Ausgangsprofil $\mathbf{y}$ gebildet. Das gemessene Istprofil $\mathbf{y}_{ist}$ setzt sich aus dem Ausgangsprofil $\mathbf{y}$ und einem prozeßbedingten Störprofil $\mathbf{y}_{stör}$ zusammen. Entsprechend der Anzahl m der Meßwertaufnehmer 4 werden das Istprofil $\mathbf{y}_{ist}$, das Sollprofil $\mathbf{y}_{soll}$, das Störprofil $\mathbf{y}_{stör}$, das Ausgangsprofil $\mathbf{y}$ und die Regelabweichung $\mathbf{e}$ jeweils durch einen m-dimensionalen Vektor mit m Größen gebildet. Das Stellprofil $\mathbf{u}$ ist in Form eines n-dimensionalen Vektors dargestellt, in dem die Stellgrößen für die n Stellglieder 3 zusammengefaßt sind. Die Charakteristik des zu regelnden Prozesses wird hier als linear angenommen und es wird nur der statische Anteil, nämlich die m×n-Übertragungsmatrix P betrachtet, die aus dem Stellprofil $\mathbf{u}$ das Ausgangsprofil $\mathbf{y}$ mit $\mathbf{y} = \mathbf{P}\mathbf{u}$ bildet.

Es ist plausibel, daß mit der endlichen Anzahl n von Stellgliedern 3 nicht jede beliebige Form für das Profil $\mathbf{y}$ eingestellt werden kann. Um die Verbesserungsfähigkeit der Querprofil-Regelung abschätzen zu können, wird zunächst in der Einheit 9 bei vorgegebenem Sollprofil $\mathbf{y}_{soll}$ und einem prozeßbedingten Störprofil $\mathbf{y}_{stör}$ das optimal erreichbare Profil, d. h. das Profil mit der kleinsten Varianz $\sigma^2$ und damit der kleinsten Standardabweichung $\sigma$ berechnet. In der Praxis wird üblicherweise die doppelte Standardabweichung als Qualitätsmerkmal für das eingestellte Profil herangezogen.

Im folgenden wird das m-dimensionale Profil der Regelabweichung

$$e = y_{soll} - (Pu + y_{stör})$$

betrachtet. Mittels $\mathbf{u}$ soll nun die Varianz

$$\sigma^2 = e^T e$$

$$= (y_{soll} - Pu - y_{stör})^T (y_{soll} - Pu - y_{stör})$$

und damit die Standardabweichung $\sigma$ minimiert werden. Die Varianz $\sigma^2$ wird dann minimal, wenn

$$\partial\sigma^2/\partial u = -2P^T(y_{soll} - y_{stör}) + 2P^T Pu_{opt} = 0$$

gilt. Hieraus resultiert als Lösung

$$u_{opt} = (P^T P)^{-1} P^T (y_{soll} - y_{stör}).$$

Für das optimale Profil $\mathbf{e}_{opt}$ ergibt sich folglich

$$e_{opt} = (I - P(P^T P)^{-1} P^T) (y_{soll} - y_{stör})$$

mit der m×n-Einheitsmatrix $\mathbf{I}$ und der Pseudoinversen

$$P^\circ = (P^T P)^{-1} P^T.$$

Mit der Beziehung

$$y_{stör} = y_{ist} - Pu$$

für das Störprofil $\mathbf{y}_{stör}$ erhält man schließlich für die im optimalfall erzielbare geringstmögliche Regelabweichung

$$e_{opt} = (I - P(P^T P)^{-1} P^T)\,(y_{soll} - y_{ist}).$$

Mit diesem Algorithmus wird in der Einheit 9 bei einem vorgegebenen Sollprofil $\mathbf{y}_{soll}$ direkt aus dem gemessenen Istprofil $\mathbf{y}_{ist}$ das im Optimalfall erzielbare bestmögliche Querprofil, d. h. das Profil mit der geringstmöglichen Varianz bzw. Standardabweichung, ermittelt. Das optimale Profil $\mathbf{e}_{opt}$ der Regelabweichung wird zusammen mit dem momentanen Profil der Regelabweichung $\mathbf{e} = \mathbf{y}_{soll} - \mathbf{y}_{ist}$ der Anzeigevorrichtung 8 zugeführt und dort bildlich dargestellt. Der direkte bildliche Vergleich zwischen dem Optimalprofil $\mathbf{e}_{opt}$ und dem momentanen Regelfehlerprofil $\mathbf{e}$ erlaubt dann eine Beurteilung der noch bestehenden Verbesserungsmöglichkeiten. Er kann aber auch dazu herangezogen werden, um zu demonstrieren, daß ein Profil nicht mehr zu verbessern ist oder daß bestimmte Zacken innerhalb des gemessenen Profils nicht mehr verkleinert werden können. Natürlich kann ein solcher Vergleich zwischen dem errechneten Optimalprofil $\mathbf{e}_{opt}$ und dem momentanen Regelfehlerprofil $\mathbf{e}$ auch automatisch in der Einheit 9 bzw. der Recheneinrichtung 5 erfolgen.

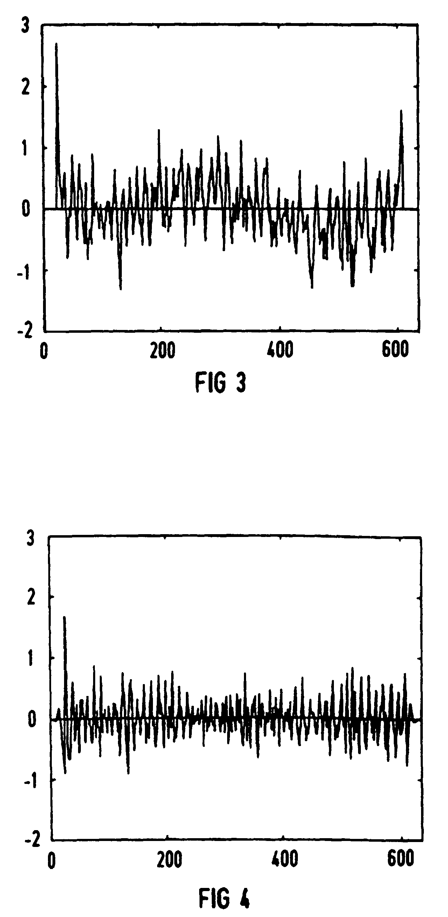

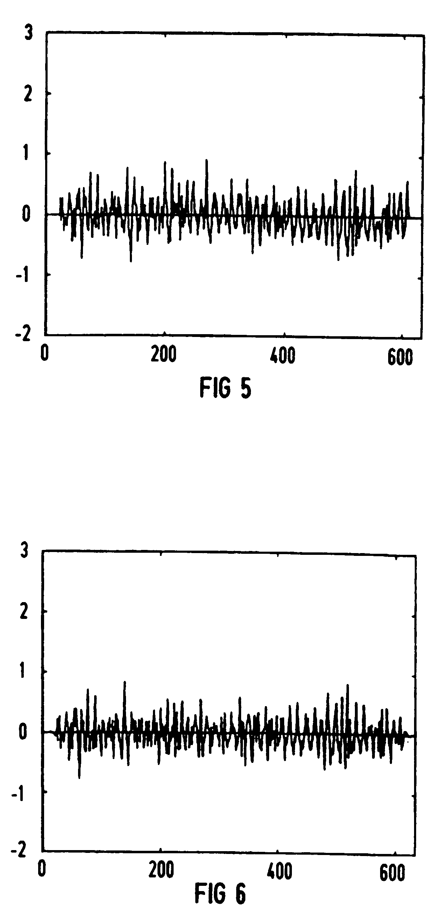

FIG 3 zeigt am Beispiel der Querprofilregelung einer Papiermaschine das vor Regelungsbeginn gemessene Flächengewicht-Querprofil. Das in der Einheit 9 berechnete zugehörige Optimalprofil ist in FIG 4 dargestellt. FIG 5 zeigt das gemessene Querprofil nach Inbetriebnahme der Querprofilregelung und FIG 6 wieder das zugehörige Optimalprofil. Die Diagramme sind in g/m² über Meßwertnummern skaliert. Ein Vergleich der FIG 3 und 4 zeigt, daß vor Inbetriebnahme der Querprofilregelung noch ein Verbesserungspotential von 58% bestand, während, wie ein Vergleich der FIG 5 und 6 zeigt, nach Inbetriebnahme der Regelung die Verbesserungsmöglichkeiten nur noch 14% betrugen; d. h. das Istprofil entspricht zu 86% dem optimal erreichbaren Profil.

Prinzipiell sind Profilanteile mit niedriger Frequenz, d. h. solche, die eine deutlich größere Wellenlänge als der Abstand zweier benachbarter Stellglieder 3 aufweisen, gut ausregelbar, wogegen höherfrequente Anteile nur schlecht oder nicht ausgeregelt werden können. Um eine Information darüber zu erhalten, wie weit die einzelnen Spektralkomponenten des Querprofils durch die Regelung im optimalen Fall verbessert werden können, werden in der Einheit 9 unter Verwendung einer Transformationsmatrix $\mathbf{T}$ mit den Elementen

$$t_{ki} = \exp(-j2\pi ki/m) \qquad , i,k = 0,1,\dots,m-1$$

die Fouriertransformierten $\mathbf{E}$ und $\mathbf{E}_{opt}$ des gemessenen Profils der Regelabweichung $\mathbf{e}$ und des Optimalprofils $\mathbf{e}_{opt}$ bestimmt:

$$E = Te$$

$$E_{opt} = Te_{opt} = T[I - P(P^T P)^{-1} P^T]T^{-1} E$$

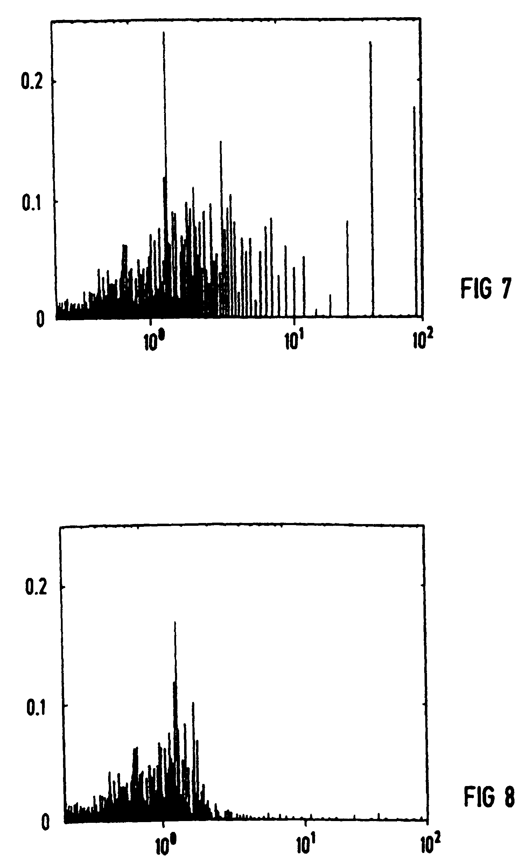

Die Bestimmung des möglichen Verbesserungspotentials des Querprofils mittels der Profilspektren $\mathbf{E}$ und $\mathbf{E}_{opt}$ erfolgt in gleicher Weise, wie dies obenstehend bereits für die Profile $\mathbf{e}$ und $\mathbf{e}_{opt}$ erläutert worden ist. Die FIG 7 bis 10 erläutern dies anhand der zu den Profilen aus den FIG 3 bis 6 gehörenden Spektren. Dabei sind die Amplituden $|E_k|$ der einzelnen Spektralkomponenten $\mathbf{E}_k$ bezüglich ihrer Wellenlänge

$$\lambda_k = n/k$$

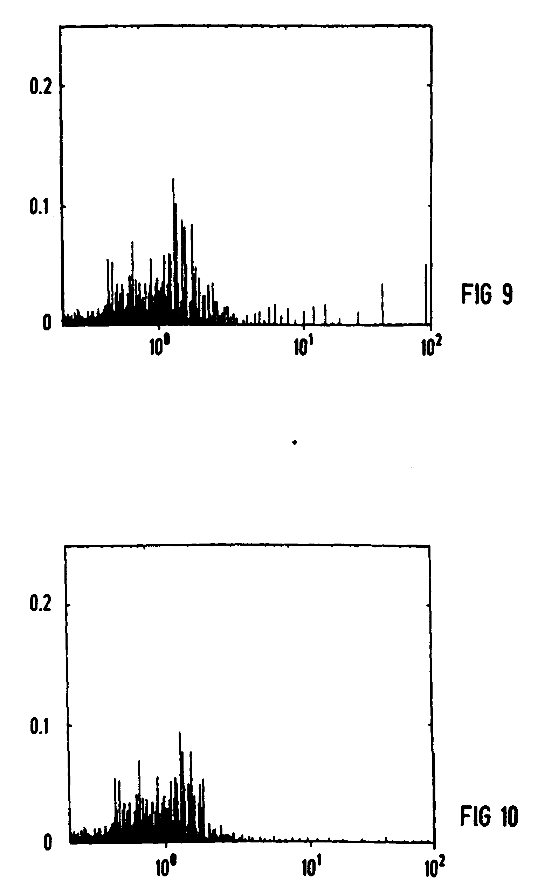

aufgetragen, wobei die Wellenlängen $\eta_k$ der einzelnen Spektralkomponenten $\mathbf{E}_k$ als Vielfache eines Stellgliedabstandes aufgetragen sind. Im einzelnen zeigen FIG 7 das Spektrum $\mathbf{E}$ des gemessenen Profils $\mathbf{e}$ vor der Regelung, FIG 8 das Spektrum $\mathbf{E}_{opt}$ des zugehörigen Optimalprofils $\mathbf{e}_{opt}$, FIG 9 das Spektrum $\mathbf{E}$ des gemessenen ausgeregelten Profils $\mathbf{e}$ und FIG 10 das Spektrum $\mathbf{E}_{opt}$ des entsprechenden Optimalprofils $\mathbf{e}_{opt}$. Wie den FIG 7 bis 10 zu entnehmen ist, wird durch die Regelung vor allem eine Verbesserung des Profils im langwelligen Bereich erzielt.

Wie vorstehend bereits festgestellt wurde, existiert zu jedem Störprofil $\mathbf{y}_{stör}$ bzw. gemessenen Profil $\mathbf{y}_{ist}$ ein

eigenes spezielles Optimalprofil $\mathbf{e}_{opt}$. Jedes dieser Optimalprofile $\mathbf{e}_{opt}$ besitzt auch eine eigene Spektralverteilung $\mathbf{E}_{opt}$, aus der zu erkennen ist, wie groß die Amplituden $|E_{opt,k}|$ der einzelnen Wellenlängen $\eta_k$ sind. Dabei führen unterschiedliche Spektralverteilungen $\mathbf{E}_{opt}$ der Optimalprofile im allgemeinen auch zu unterschiedlichen Amplitudenwerten $|E_{opt,k}|$. Eine wichtige Kenngröße ist dann der größte mögliche Wert von $|E_{opt,k}|$, also seine obere Grenze $\eta_k$. Sie gibt an, wie schlecht die Optimallösung für die Wellenlänge $\lambda_k$ maximal sein kann bzw. wie gut sie mindestens sein muß. Die Grenze $\eta_k$ gibt an, mit welchem Faktor die Amplitude $|E_k|$ einer Störung der Wellenlänge $\lambda_k$ mindestens reduziert werden kann. Die Berechnung der Grenze $\eta_k$ erfolgt in der Einheit 9, wobei man als Ergebnis erhält

$$\eta_k \;=\; \max_{\|E\|_2=1} |E_{opt,k}| \;=\; (1-1/m\cdot\|P(P^TP)^{-1}P^Tt_k\|_2^2)^{1/2}.$$

Dabei bezeichnet $t_k$ den k-ten Spaltenvektor der Transformationsmatrix $\mathbf{T}$. Zusammen bilden die Grenzwerte $\eta_k$ die Grenzkurve

$$\eta^T = (\eta_0,\eta_1,...,\eta_{m-1}),$$

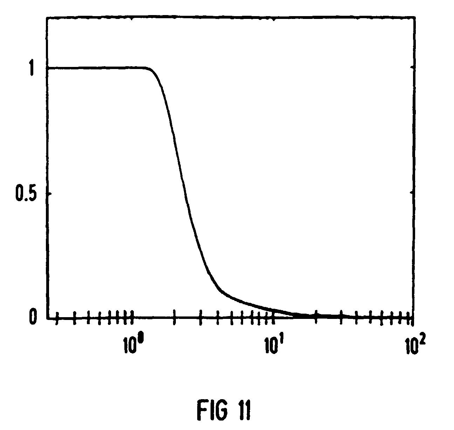

die nicht mehr von einzelnen Profilen, sondern nur noch von der Charakteristik $\mathbf{P}$ der Regelstrecke 11 abhängig ist. Ist $\mathbf{P}$ konstant, so muß die Grenzkurve $\eta$ nur einmal bestimmt werden. Schwankt $\mathbf{P}$ dagegen - z.B. in Abhängigkeit vom Prozeßzustand -, so ist in der Regel eine entsprechende Neubestimmung der Grenzkurve $\eta$ vorzunehmen. FIG 11 zeigt ein Beispiel für den Verlauf der Grenzkurve $\eta$ in Abhängigkeit von den Wellenlängen $\lambda_k$, die als Vielfache des Stellgliedabstandes angegeben sind. Es ist deutlich erkennbar, daß für Wellenlängen $\lambda_k$ unter zwei Stellgliedabständen kaum eine Verbesserung erzielbar ist.

**Patentansprüche**

1.   Verfahren zur Untersuchung des Querprofils einer kontinuierlich erzeugten Materialbahn (1), wobei für eine Querprofilregelung mit einer Regeleinrichtung (10), der eingangsseitig die Regelabweichung (**e**) zwischen einem vorgegebenen Sollprofil ($\mathbf{y}_{soll}$) und dem gemessenen Profil ($\mathbf{y}_{ist}$) der Materialbahn (1) zuführbar ist und die ausgangsseitig in Abhängigkeit von der Regelabweichung (**e**) eine vorgegebene Anzahl von Stellgliedern (3) zum Einstellen des Profils (y) der Materialbahn (1) betätigen kann, in einer Recheneinrichtung (5, 9) in Abhängigkeit von einem mathematischen Modell der Regelstrecke (11) Querprofile berechenbar sind, **dadurch gekennzeichnet**, daß in der Recheneinrichtung (5, 9) das durch eine Regelung, die das gemessene Profil ($\mathbf{Y}_{ist}$) und das mathematische Modell berücksichtigt, optimal erreichbare Profil ermittelt und daß das ermittelte Profil für einen Vergleich mit dem gemessenen Profil ($\mathbf{Y}_{ist}$) zur Verfügung gestellt wird.

2.   Verfahren nach Anspruch 1, **dadurch gekennzeichnet**, daß zur Ermittlung des optimal erreichbaren Profils das Profil der minimal erreichbaren Regelabweichung ($\mathbf{e}_{opt} = (\mathbf{I} - \mathbf{P}(\mathbf{P}^T\mathbf{P})^{-1}\mathbf{P}^T)\,(\mathbf{y}_{soll}-\mathbf{y}_{ist})$) berechnet wird, wobei (**P**) die Übertragungsmatrix der Regelstrecke (11) bezeichnet, die aus den Stellgrößen (**u**) der Stellglieder (3) das Querprofil (**y**) der Materialbahn (1) gemäß ($\mathbf{y} = \mathbf{Pu}$) erzeugt, und wobei ($\mathbf{P}^T$) die entsprechende transponierte Matrix und (**I**) die Einheitsmatrix bezeichnen.

3.   Verfahren nach Anspruch 1, **dadurch gekennzeichnet**, daß zur Ermittlung des optimal erreichbaren Profils die Fouriertransformierte des Profils der minimal erreichbaren Regelabweichung ($\mathbf{E}_{opt} = \mathbf{T}[\mathbf{I}-\mathbf{P}(\mathbf{P}^T\mathbf{P})^{-1}\mathbf{P}^T]\mathbf{T}^{-1}\mathbf{E}$) berechnet wird, wobei (**P**) die Übertragungsmatrix der Regelstrecke (11) bezeichnet, die aus den Stellgrößen (**u**) der Stellglieder (3) das Querprofil (**y**) der Materialbahn (1) gemäß ($\mathbf{y} = \mathbf{Pu}$) erzeugt, und wobei ($\mathbf{P}^T$) die entsprechende transponierte Matrix, (**I**) die Einheitsmatrix und (**T**) die Transformationsmatrix für die Fouriertransformation bezeichnen.

4.   Verfahren nach Anspruch 3, **dadurch gekennzeichnet**, daß in der Recheneinrichtung (5, 9) eine spektrale Grenzkurve ($\eta^T = (\eta_0,\eta_1,...,\eta_{m-1})$) mit

$$(\eta_k \;=\; (1-1/m\cdot\|P(P^TP)^{-1}P^Tt_k\|_2^2)^{1/2})$$

berechnet wird, die unabhängig von der momentanen Regelabweichung (**e**) für jede Wellenlänge ($\lambda_k$) die jeweils größten möglichen Amplituden ($|E_{opt,k}|$) des Fourierspektrums (**E**$_{opt}$) des Profils der minimal erreichbaren Regelabweichung (**e**$_{opt}$) angibt, wobei (m) die Anzahl der Spaltenvektoren und (**t**$_k$) den k-ten Spaltenvektor der Transformationsmatrix (**T**) bezeichnen.

5. Verfahren nach einem der vorangehenden Ansprüche, **dadurch gekennzeichnet**, daß das gemessene Profil (**e**) und das ermittelte optimal erreichbare Profil (**e**$_{opt}$) bzw. deren Fouriertransformierten (**E**, **E**$_{opt}$) auf einer Anzeigevorrichtung (8) bildlich dargestellt werden.

## Claims

1. Method for examining the transverse profile of a continuously produced material web (1), wherein, for a transverse profile control with a control device (10), to which there can be supplied on the input side the system deviation (**e**) between a specified reference profile (**y**$_{ref}$) and the measured profile (**y**$_{act}$) of the material web (1), and which can operate on the output side in dependence upon the system deviation (**e**) a specified number of actuators (3) for adjusting the profile (**y**) of the material web (1), transverse profiles can be calculated in a computing device (5, 9) in dependence upon a mathematical model of the controlled system (11), characterized in that in the computing device (5, 9) that profile is determined which is optimally achievable by a control which considers the measured profile (**Y**$_{act}$) and the mathematical model, and in that the determined profile is made available for a comparison with the measured profile (**Y**$_{act}$).

2. Method according to claim 1, characterized in that in order to determine the optimally achievable profile the profile of the minimally achievable system deviation (**e**$_{opt}$ = (**I** - **P**(**P**$^T$**P**)$^{-1}$**P**$^T$) (**y**$_{ref}$-**y**$_{act}$)) is calculated, wherein (**P**) denotes the transfer matrix of the controlled system (11) which produces from the manipulated variables (**u**) of the actuators (3) the transverse profile (**y**) of the material web (1) according to (**y** = **Pu**), and wherein (**P**$^T$) denotes the corresponding transposed matrix and (**I**) the unit matrix.

3. Method according to claim 1, characterized in that in order to determine the optimally achievable profile the Fourier transform of the profile of the minimally achievable system deviation (**E**$_{opt}$ = **T**[**I**-**P**(**P**$^T$**P**)$^{-1}$**P**$^T$]**T**$^{-1}$**E**) is calculated, wherein (**P**) denotes the transfer matrix of the controlled system (11) which produces from the manipulated variables (**u**) of the actuators (3) the transverse profile (**y**) of the material web (1) according to (**y** = **Pu**), and wherein (**P**$^T$) denotes the corresponding transposed matrix, (**I**) the unit matrix and (**T**) the transformation matrix for the Fourier transformation.

4. Method according to claim 3, characterized in that in the computing device (5, 9) a spectral limiting curve ($\eta^T$ = ($\eta_0,\eta_1,...,\eta_{m-1}$)) with

$$( \eta_k = (1-1/m \cdot \| P(P^T P)^{-1} P^T t_k \|_2^2)^{1/2})$$

is calculated, which provides, for any wavelength ($\lambda_k$), the respectively largest possible amplitudes ($| E_{opt,k}|$) of the Fourier spectrum (**E**$_{opt}$) of the profile of the minimally achievable system deviation (**e**$_{opt}$) independently of the instantaneous system deviation (**e**), wherein (m) denotes the number of column vectors and (**t**$_k$) denotes the kth column vector of the transformation matrix (**T**).

5. Method according to one of the preceding claims, characterized in that the measured profile (**e**) and the determined optimally achievable profile (**e**$_{opt}$) or their Fourier transforms (**E**, **E**$_{opt}$) are graphically represented on a display device (8).

## Revendications

1. Procédé d'analyse du profil transversal d'une carte (1) de matériau produite de manière continue, dans lequel pour une régulation du profil transversal par un dispositif (10) de régulation, auquel en entrée on peut envoyer les écarts (e) de régulation entre un profil (y$_{soll}$) de consigne donné à l'avance et le profil (y$_{ist}$) mesuré de la bande (1) de matériau et qui, en sortie, peut actionner, en fonction des écarts (e) de régulation, un nombre donné à l'avance d'actionneurs (3) pour régler le profil (y) de la bande (1) de matériau, on peut calculer des profils transversaux, dans un dispositif (5, 9) de calcul, en fonction d'un modèle mathématique du système (11) réglé, caractérisé en ce que dans le dispositif (5, 9) de calcul on détermine par une régulation, qui prend en compte le profil ($y_{ist}$) mesuré et le modèle mathématique, le profil que l'on peut atteindre de manière optimale et en ce que le profil déterminé est mis à disposition pour une comparaison avec le profil ($y_{ist}$) mesuré.

2. Procédé suivant la revendication 1, caractérisé en ce que pour la détermination du profil optimal pouvant être obtenu, on calcule le profil de l'écart de régulation minimal pouvant être obtenu ($e_{opt} = (I - P(P^TP)^{-1} P^T) (y_{soll} - y_{ist})$), (P) désignant la matrice de transfert du système (11) réglé, qui produit à partir des grandeurs (u) de réglage des actionneurs (3) le profil (y) transversal de la bande (1) de matériau suivant ($y = Pu$), ($P^T$) désignant la matrice transposée correspondante et (I) la matrice unité.

3. Procédé suivant la revendication 1, caractérisé en ce que, pour la détermination du profil optimum pouvant être obtenu, on calcule la transformée de Fourier du profil de l'écart de régulation minimum pouvant être obtenu ($E_{opt} = T[I-P(P^TP)^{-1}P^T]T^{-1}E$) où (P) représente la matrice de transfert du système (11) réglé, qui produit à partir des grandeurs (u) de réglage des actionneurs (3) le profil (y) transversal de bande (1) de matériau suivant ($y = Pu$), ($P^T$) représentant la matrice transposée correspondante, (I) représentant la matrice unitaire et (T) la matrice de transformation pour la transformation de Fourier.

4. Procédé suivant la revendication 3, caractérisé en ce que dans le dispositif (5, 9) de calcul, il est calculé une courbe limite spectrale ($\eta^T = (\eta_0, \eta_1, ..., \eta_{m-1})$) avec ($\eta_k = (1-1/m \|P(P^TP)^{-1}P^Tt_k\|^2)^{1/2}$), qui donne indépendamment de l'écart (e) de régulation instantané pour chaque longueur d'ondes ($\lambda_k$) les amplitudes respectives ($IE_{opt,k}I$) les plus grandes possibles du spectre ($E_{opt}$) de Fourier du profil de l'écart ($e_{opt}$) de régulation minimum pouvant être obtenu, (m) représentant le nombre de vecteurs de colonnes et ($t_k$) le vecteur de colonne numéro k de la matrice (T) de transformation.

5. Procédé suivant l'une des revendications précédentes, caractérisé en ce que le profil (e) mesuré et le profil ($e_{opt}$) optimal pouvant être obtenu qui est déterminé ou leur transformée de Fourier (E, $E_{opt}$) sont représentés en image sur un dispositif (8) d'affichage.

FIG 1

FIG 2

FIG 3

FIG 4

FIG 5

FIG 6

FIG 7

FIG 8

FIG 9

FIG 10

FIG 11